# EUROPEAN PATENT APPLICATION

(11) **EP 2 213 305 A1**
(43) Date of publication of application: **04.08.2010**
(21) Application number: 08855278.1
(22) Date of filing: 25.11.2008
(51) Int. Cl.: A61K 47/02, A61K 9/14, A61K 9/32, A61K 9/50, A61K 31/4433, A61K 31/4439, A61K 47/26, A61K 47/32, A61K 47/36, A61K 47/38, A61P 1/04, A61P 43/00

(54) **METHOD FOR PRODUCING GRANULATED MATTER**

(30) Priority: 27.11.2007 JP 2007306466
(71) Applicant: Ohara Pharmaceutical Co., Ltd., Shiga 520-3433 (JP)
(72) Inventor: SAKAMOTO, Hiroshi, Sakai-shi Osaka 590-0134 (JP); TANIGUCHI, Toshiya, Kouka-shi Shiga 520-3413 (JP)
(74) Representative: von Kreisler Selting Werner
(86) International application number: PCT/JP2008/003450
(87) International publication number: WO 2009/069280

(57) **Abstract**

It has been desired to provide a method for producing a granulated preparation capable of maintaining a stability of a chemically unstable substance in a neutral or acidic region for a long period of time with a simple and safe method in a preparation procedure, and a tablet produced by using the method.
The invention provides a granulation method in which an unstable substance is successively subjected to aqueous stabilization treatment and granulation procedure. Further, it became possible to provide a tablet which is absorbed in the intestine without losing the potency in a gastric region by forming an intermediate layer on a surface of the thus obtained granule and subsequently subjecting the granule to enteric coating.

## Description

### TECHNICAL FIELD

The present invention relates to a method for producing a granulated preparation and particularly to a method for producing a granulated preparation containing an unstable substance.

### BACKGROUND ART

Some substances may become chemically unstable due to moisture, pH of the solution, heat, or incorporation or contact with other materials and, as a result, decomposition, deactivation, or the like may occur, wherein such substances are referred hereinafter to as "unstable substances". In particle processing (e.g. granulation-coating and tableting) of such a substance, which becomes unstable under some conditions, choice of conditions which have no effect on the stability of the substance requires a great deal of time, labor, and expenses. Moreover, keeping the quality of such processed granular stable needs a more complicated particle processing method and satisfaction of the need greatly lowers the production efficiency of a granulated preparation and requires high processing fees.

According to a fluidized bed granulation method that has been used widely, a raw powder charged into a fluidized bed granulator is fluidized by fluidization air blown through an air passage provided at the bottom of the apparatus, and a mist sprayed here through a spray nozzle for a binder liquid provided at an upper portion of the fluidized bed adheres to the surface of the raw powder and comes into contact with particles that float and flow near, so that adhesion or aggregation of particles proceeds. More specifically, a mist of a binder liquid (e.g., an aqueous solution of hydroxypropylcellulose or an alcoholic solution of hydroxypropylcellulose) adheres to a floating-flowing substance and an excipient, and adhesion and aggregation of particles proceed, so that granules are formed gradually and a nuclear substance suitable for coating in the following steps is obtained.

However, if the substance in a fluidized state is a substance unstable in an acid and the mist of the aqueous solution of the binder sprayed and added through the nozzle is acidic, the unstable substance decomposes and deteriorates through contact or reacting to the water in the aqueous solution. Although when an unstable substance, a stabilizing agent and an excipient which causes the unstable substance to be unstable are compounded and granulated under stirring, then kneaded by the addition of a binder liquid and stirring mechanically, the unstable substance adheres to the excipient at a higher probability rather than the unstable substance and the stabilizing agent adhere and combine together. In this case, destabilization of the unstable substance goes ahead, and then the destabilized substance contacts to the stabilizing agent. A similar phenomenon occurs also in fluidized bed granulation.
Although ethanol may be used as a solvent of a spray liquid, ethanol is more expensive than water and since it is a combustible solvent, it has a high risk of ignition-explosion, and the like and it produces a need to make a facility explosion-proof. Moreover, discharge of a combustible solvent is undesirable in view of recent global environmental problems. In this sense, water is preferred as a solvent of a spray liquid.

As to specific examples of conventional technologies, methods for producing, by an ordinary method, a nuclear substance in which an unstable substance and a stabilizing agent have been blended have been disclosed (patent documents 1 and 2). Moreover, it has been proposed that in order to prevent deterioration in quality of an unstable agent in the production of a nuclear material suitable for coating in the following steps, an unstable substance is fluidized alone or with incorporation of a stabilizing agent powder without incorporation of any other powders or particles and then an aqueous solution in which the unstable substance has been dissolved is sprayed and added thereto (patent document 3). However, that the unstable substance is unstable in water is inconvenient because the destabilization of the substance proceeds if it is dissolved in water.

Patent document 1: JP 7-68125 B
Patent document 2: JP 2000-355540 A
Patent document 3: JP 2006-131548 A

### DISCLOSURE OF THE INVENTION

An object of the present invention is to provide a method for granulating a substance chemically unstable in a neutral or acidic region to form granules stable over a long term by a method with simple granulation operations.

As a result of hard and extensive studies to achieve the object, the present inventors found that a stable granulated preparation could be obtained by dissolving an unstable substance in an aqueous solution or aqueous suspension of a stabilizing agent and spraying the solution to particles of a stabilizing agent or excipient in a fluidized state, and they did further studies, accomplishing the present invention.

That is, the present invention provides:
(1) A method for producing a granulated preparation, which comprises supplying particles of a stabilizing agent or an excipient to a fluidized bed granulator, and spraying a liquid in which an unstable substance has been dissolved or suspended in an aqueous solution or aqueous suspension of the stabilizing agent while keeping a fluidized state.
(2) The method for producing a granulated preparation according to (1), wherein the stabilizing agent is an alkaline substance and the unstable substance is a substance unstable under a neutral or acidic condition.
(3) The method for producing a granulated preparation according to (1) or (2), wherein the stabilizing agent is a hydroxide, oxide or carbonate of an alkali metal or alkaline earth metal, and the unstable substance is a proton pump inhibitor.
(4) The method for producing a granulated preparation according to any one of (1) to (3), wherein the stabilizing agent is sodium hydroxide, magnesium oxide, potassium carbonate, sodium carbonate, or potassium hydroxide and the unstable substance is sodium rabeprazole, omeprazole, or lansoprazole.
(5) The method for producing a granulated preparation according to any one of (1) to (4), wherein the amount of the stabilizing agent in the aqueous solution or the aqueous suspension of the stabilizing agent is 0.01 to 10% by weight relative to the granulated preparation.
(6) The method for producing a granulated preparation according to any one of (1) to (5), wherein the concentration of the stabilizing agent in the aqueous solution or the aqueous suspension of the stabilizing agent is 0.1 to 33% by weight.
(7) The method for producing a granulated preparation according to any one of (1) to (6), wherein a surface of the excipient has been subjected to stabilizing treatment with a stabilizing agent.
(8) The method for producing a granulated preparation according to any one of (1) to (7), wherein the excipient is lactose, crystalline cellulose, corn starch, potato starch, partially pregelatinized starch, D-mannitol, white soft sugar, sucrose, glucose, light silicic anhydride, calcium silicate, or sodium carboxymethylstarch.
(9) A method for producing a granulated preparation, which comprises forming a monolayer or multilayer film by using an aqueous macromolecular film-forming agent liquid in which a stabilizing agent or another additive has been dissolved or suspended, on the surfaces of granules prepared by the granulation method according to any one of (1) to (8), and subsequently coating the granules with film-forming agent for elution control.
(10) The method for producing a granulated preparation according to (9), wherein the macromolecular compound of the macromolecular film forming agent is at least one member selected from the group consisting of hydroxypropylcellulose, hydroxypropylmethylcellulose, polyvinyl pyrrolidone, polyvinyl alcohol, polyvinyl alcohol copolymer, aminoalkyl methacrylate copolymer (E, RS), methacrylic acid copolymer (L, LD, S), methylcellulose, hydroxypropyl, methylcellulose phthalate, hydroxypropylmethylcellulose acetate succinate, and ethylcellulose compounds.
(11) A method for producing a tablet, which comprises mixing granules prepared by the granulation method according to (9) or (10) with another additive, and compressing them to make tablets.
(12) A method for producing a tablet, which comprises mixing granules prepared by the granulation method according to any one of (1) to (8) with another additive, compressing them, then forming a monolayer or multilayer film by using an aqueous macromolecular film-forming agent liquid in which a stabilizing agent or another additive has been dissolved or suspended, and subsequently coating a tablet with a film-forming agent for elution control.

The stabilizing agent in the present invention is a substance that can hold an unstable substance under certain conditions stable, and examples thereof include alkaline substances for substances unstable in a neutral or acidic region.
The alkaline substances include, for example, hydroxides, oxides, or carbonates of alkali metals or alkaline earth metals. Specific examples include sodium hydroxide, magnesium oxide, potassium carbonate, sodium carbonate, and potassium hydroxide. Among them sodium hydroxide is preferable. The use amount of the stabilizing agent in the aqueous solution or aqueous suspension of the stabilizing agent is 0.01 to 10% by weight, preferably 0.05 to 7.5% by weight, and more preferably 0.1 to 5% by weight based on the whole weight of the granulated preparation. The concentration of the stabilizing agent in the aqueous solution or aqueous suspension of the stabilizing agent is 0.1 to 33% by weight, preferably 0.5 to 25% by weight, and more preferably 1 to 20% by weight.

When the unstable substance is a substance unstable under a neutral or acidic condition, the substance is, for example, a proton pump inhibitor, which is important as a drug. The typical examples of the proton pump inhibitor include sodium rabeprazole, omeprazole, and lansoprazole. The amount of the unstable substance in a liquid prepared by dissolving or suspending the unstable substance in an aqueous solution or aqueous suspension of the stabilizing agent is 0.5 to 95% by weight, preferably 1 to 90% by weight, and more preferably 5 to 85% by weight based on the whole weight of the granulated preparation (stabilized uniform granules) obtained by spraying a liquid prepared by dissolving or suspending the unstable substance in a aqueous solution or aqueous suspension of the stabilizing agent to stabilizing agent particles or an excipient.

A fluidized bed granulator usually is composed of a fluidized bed body, a distributor, a blower, an air filter, a heat exchanger, a spray device, a dust collector, an exhauster, etc. Although the air supplied from the air blasting fan is cleaned with the air filter, warmed with the heat exchanger, and blown into the main body of the apparatus through the distributor, the hot air keeps the powder of a stabilizing substance or an excipient having been charged into the apparatus in a suspension state, i.e., a fluidized state. By spraying a solution prepared by dissolving an unstable substance in an aqueous solution of a stabilizing agent in the form of a mist, the mist is adhered to the surface of the unstable substance as a binder to modify the surface (stabilizing treatment), and at the same time fine particles of the unstable substance undergo adhesion-aggregation repeatedly through the binder mist. Thus, particle growth by granulation and coating proceeds gradually.

Examples of the excipient, which is sometimes referred to as a vehicle, include lactose, crystalline cellulose, corn starch, potato starch, pregelatinized starch, D-mannitol, white soft sugar, sucrose, glucose, low-substituted hydroxypropylcellulose, light silicic anhydride, calcium silicate, or sodium carboxymethylstarch. Such excipients may be dissolved or dispersed and suspended partly or wholly in a solution of a binder. Moreover, it is desirable that such excipients are used after being subjected on their surfaces to stabilizing treatment (surface modification) by a treating method, such as spraying a solution of a stabilizing agent.

In the granulation method of the present invention, the stabilizing agent powder and the excipient are used as seeds in granulation. The particle size of the seed is about 0.1 to about 200 µm in average particle diameter.

Examples of the macromolecular compound of the aqueous macromolecular film forming agent used in the present invention include hydroxypropylcellulose, hydroxypropylmethylcellulose, ethylcellulose, methylcellulose, polyvinyl pyrrolidone, polyvinyl alcohol, and polyvinyl alcohol copolymers. In particular, hydroxypropylmethylcellulose, polyvinyl alcohol, and polyvinyl alcohol copolymers are preferable. Furthermore, additives, such as talc, may be incorporated. The aforesaid polyvinyl alcohol copolymer means a polyvinyl alcohol-acrylic acid-methyl methacrylate copolymer, and particularly means a copolymer obtained by copolymerizing a partially saponified polyvinyl alcohol having an average polymerization degree of 300 to 3000, methyl methacrylate, and acrylic acid at weight ratios of 60 to 90 : 7 to 38 : 0.5 to 12. The use amount of such an aqueous macromolecular film forming agent is preferably 1 to 50% by weight, more preferably 3 to 30% by weight of the whole weight of the tablet. In addition, additives usually used, such as an excipient, a disintegrator, a binder, a corrigent, a colorant, and a tonicity adjusting agent, may be used appropriately as additives capable of being used in preparation.

Examples of a film forming agent for elution control include conventional enteric film agents, such as aminoalkyl methacrylate copolymer (E, RS), methacrylic acid copolymer (L, LD, S), METOLOSE (Trade name of methylcellulose), and hydroxypropyl methylcellulose phthalate, hydroxypropylmethylcellulose acetate succinate, and ethylcellulose based aqueous dispersion. The film forming agent may be incorporated either alone or in combination. Examples of the disintegrator include carboxymethylcellulose, calcium carboxymethylcellulose, sodium carboxymethylstarch, crospovidone, low-substituted hydroxypropylcellulose, and pregelatinized starch.

By covering or concealing the particle surface with a mist of an aqueous solution of a stabilizing agent, it is possible to prevent the decomposition-deterioration of the particle even if mixing it with other additives, such as an excipient. By forming an intermediate layer coating in which a stabilizing agent has been incorporated for increasing an effect, it is possible to inhibit the moisture in the air from being adsorbed. Subsequently, by applying a film forming agent for elution control, such as an enteric coating, it is also possible to prevent the deterioration or deactivation of the unstable substance caused by direct contact of the enteric film forming agent with a drug. Examples of the enteric film forming agent include conventional agents, such as methacrylic acid copolymer, cellulose acetate phthalate, and hydroxypropylmethylcellulose phthalate. Moreover, a stabilizing agent may be added for adjusting the pH of the enteric film forming agent.

The concrete method for surface modification of the unstable substance in the present invention is not particularly restricted. For example, by spraying an aqueous solution of a stabilizing agent, such as sodium hydroxide as a binder on the surface of fine particles of the unstable substance in a fluidized state, a mist from a spray nozzle is adhered to the surfaces of the particles of the unstable substance to perform surface modification (stabilizing treatment) and the fine particles undergo adhesion-aggregation repeatedly through the binder mist. Through these steps including granulation and coating, the particles grow gradually. Furthermore, an intermediate layer coating layer may be formed for the purpose of preventing the surface of the granulated preparation of the drug having been subjected to stabilizing treatment (surface modification) from undergoing contact or interference with an enteric film forming agent and the purpose of inhibiting the adsorption of the moisture in the air.

### BEST MODE FOR CARRYING OUT THE INVENTION

The present invention is hereinafter described concretely with examples.

### Example 1

356.0 g of magnesium oxide was charged into a jet fluidized bed granulator (Model MP-01-SPC, manufactured by Powrex Corporation) and then was fluidized. Granules were produced by spraying a liquid prepared by dissolving 80.0 g of sodium rabeprazole in a liquid prepared by dissolving 20.0 g of sodium hydroxide in 220.0 g of purified water to the fluidized bed and then were dried. The granules were made uniform in size by being passed through a sieve of JIS 24 mesh (stabilized uniformized granules). 114.0 g of the resulting stabilized uniformized granules were charged into a jet fluidized bed granulator, and the granules were provided with coating by spraying a liquid prepared by dissolving and suspending 11.0 g of polyvinyl alcohol copolymer and 11.0 g of talc in 253.0 g of purified water, and then were dried. Subsequently, the granules were provided with coating by spraying a liquid prepared by dissolving and suspending 62.0 g of methacrylic acid copolymer LD (30% by weight liquid), 9.6 g of talc, and 1.8 g of triethyl citrate in purified water, and then were dried. A resulting coated product and 794.0 g of D-mannitol were charged into a jet fluidized bed granulator, and granules were produced by spraying a liquid prepared by dissolving 40.0 g of hydroxypropylcellulose in 760 g of purified water and then were dried. Thus, an enteric granulated preparation having the composition given below was obtained.

| [Ingredients] | [Weight (mg) in 500 mg granulated preparation] |
|---|---|
| Sodium rabeprazole | 10.0 |
| Magnesium oxide | 44.5 |
| Sodium hydroxide | 2.5 |
| Polyvinyl alcohol copolymer | 5.5 |
| Talc | 10.3 |
| Methacrylic acid copolymer LD | 9.3 |
| Triethyl citrate | 0.9 |
| D-mannitol | 397.0 |
| Hydroxypropylcellulose | 20.0 |

### Example 2

Into the same jet fluidized bed granulator as that used in Example 1 was charged 406.0 g of D-mannitol, which was then fluidized. Subsequently, granules were produced by spraying a liquid prepared by dissolving 40.0 g of sodium rabeprazole with a mean particle diameter of 5 µm in a liquid prepared by dissolving 10.0 g of sodium hydroxide in 110.0 g of purified water and then were dried. The granules were made uniform in size by being passed through a sieve of JIS 24 mesh (stabilized uniformized granules). 228.0 g of the resulting uniformized granules were charged into a jet fluidized bed granulator, and the granules were provided with coating by using a liquid prepared by dissolving and suspending 22.0 g of polyvinyl alcohol and 22.0 g of talc in 506.0 g of purified water, and then were dried. Subsequently, the granules were provided with coating by using a liquid prepared by dissolving and suspending 124.0 g of methacrylic acid copolymer LD (30 W/W% liquid), 19.2 g of talc, and 3.6 g of triethyl citrate in purified water, and then were dried. A resulting coated product and 628.0 g of D-mannitol were charged into a jet fluidized bed granulator, and granules were produced by using a liquid prepared by dissolving 40.0 g of hydroxypropylcellulose in 760 g of purified water and then were dried. Thus, an enteric granulated preparation having the composition given below was obtained.

| [Ingredients] | [Weight (mg) in 500 mg granulated preparation] |
|---|---|
| Sodium rabeprazole | 10.0 |
| D-mannitol | 101.5 |
| Sodium hydroxide | 2.5 |
| Polyvinyl alcohol | 11.0 |
| Talc | 20.6 |
| Methacrylic acid copolymer LD | 18.6 |
| Triethyl citrate | 1.8 |
| D-mannitol | 314.0 |
| Hydroxypropylcellulose | 20.0 |

### Example 3

Into the same jet fluidized bed granulator as that used in Example 1 was charged 414.0 g of D-mannitol. Subsequently, granules were produced by using a liquid prepared by dissolving 40.0 g of sodium rabeprazole with a mean particle diameter of 5 µm in a liquid prepared by dissolving 2.0 g of sodium hydroxide in 110.0 g of purified water and then were dried. The granules were made uniform in size by being passed through a sieve of JIS 24 mesh (stabilized uniformized granules). As a result of the following operations performed in the same manner as in Example 1, an enteric granulated preparation having the composition given below was obtained.

| [Ingredients] | [Weight (mg) in 500 mg granulated preparation] |
|---|---|
| Sodium rabeprazole | 10.0 |
| D-mannitol | 103.5 |
| Sodium hydroxide | 0.5 |
| Polyvinyl alcohol copolymer | 11.0 |
| Talc | 20.6 |
| Methacrylic acid copolymer LD | 18.6 |
| Triethyl citrate | 1.8 |
| D-mannitol | 314.0 |
| Hydroxypropylcellulose | 20.0 |

### Example 4

Into 57.0 g of the stabilized uniformized granules obtained in Example 1 were added 57.0 g of D-mannitol, 5.0 g of low-substituted hydroxypropylcellulose, and 1.0 g of magnesium stearate, followed by uniformly mixing with a tumbler mixer (model TM-2S, manufactured by Syowa Giken). The mixture was compression molded with a rotary tabletizer (model VIGO, manufactured by Kikusui Seisakusho Ltd.) to yield tablets having a weight of 120 mg containing 10 mg of sodium rabeprazole per tablet. To a tablet coater (model DRC-200, manufactured by Powrex Corporation) were charged 240 g of the obtained tablets, which were then provided with coating in an amount of 5 mg per tablet by using a liquid prepared by dissolving and suspending 5.0 g of polyvinyl alcohol copolymer and 5.0 g of talc in 115.0 g of purified water and subsequently were dried. After the drying, coating was provided in an amount of 5 mg per tablet by using a liquid prepared by dissolving and suspending methacrylic acid copolymer LD, talc and triethyl citrate, followed by drying. Thus, an enteric granulated preparation having the composition given below was obtained.

| [Ingredients] | [Weight (mg) in one tablet] |
|---|---|
| Sodium rabeprazole | 10.0 |
| Magnesium oxide | 44.5 |
| Sodium hydroxide | 2.5 |
| D-mannitol | 57.0 |
| Low-substituted hydroxypropylcellulose | 5.0 |
| Magnesium stearate | 1.0 |
| Polyvinyl alcohol copolymer | 2.5 |
| Talc | 4.1 |
| Methacrylic acid copolymer LD | 3.1 |
| Triethyl citrate | 0.3 |

### Example 5

The stabilized uniformized granules obtained in Example 2 were taken in an amount of 114.0 g, and as a result of the following operations performed in the same manner as in Example 4, enteric tablets having the composition given below were obtained.

| [Ingredients] | [Weight (mg) in one tablet] |
|---|---|
| Sodium rabeprazole | 10.0 |
| D-mannitol | 101.5 |
| Sodium hydroxide | 2.5 |
| Low-substituted hydroxypropylcellulose | 5.0 |
| Magnesium stearate | 1.0 |
| Polyvinyl alcohol | 2.5 |
| Talc | 4.1 |
| Methacrylic acid copolymer LD | 3.1 |
| Triethyl citrate | 0.3 |

### Example 6

The stabilized uniformized granules obtained in Example 3 were taken in an amount of 114.0 g, and as a result of the following operations performed in the same manner as in Example 4, enteric tablets having the composition given below were obtained.

| [Ingredients] | [Weight (mg) in one tablet] |
|---|---|
| Sodium rabeprazole | 10.0 |
| D-mannitol | 103.5 |
| Sodium hydroxide | 0.5 |
| Low-substituted hydroxypropylcellulose | 5.0 |
| Magnesium stearate | 1.0 |
| Polyvinyl alcohol copolymer | 2.5 |
| Talc | 4.1 |
| Methacrylic acid copolymer LD | 3.1 |
| Triethyl citrate | 0.3 |

### Example 7

Into the same jet fluidized bed granulator as that used in Example 1 was charged 100.0 g of calcium silicate. Subsequently, granules were produced by using a liquid prepared by dissolving 200.0 g of sodium rabeprazole in a liquid prepared by dissolving 20.0 g of sodium hydroxide in 480.0 g of purified water and then were dried. The granules were made uniform in size by being passed through a sieve of JIS 24 mesh (stabilized uniformized granules). 160.0 g of the resulting stabilized uniformized granules were charged into a jet fluidized bed granulator, and the granules were provided with coating by using a liquid prepared by dissolving and suspending 120.0 g of polyvinyl alcohol copolymer and 120.0 g of talc in 2760.0 g of purified water, and then were dried. Subsequently, the granules were provided with coating by using a liquid prepared by dissolving and suspending 1550.0 g of methacrylic acid copolymer LD (30% by weight liquid), 240.0 g of talc, and 450.0 g of triethyl citrate in purified water, and then were dried. 230.0 g of the resulting coated product and 730.0 g of D-mannitol were charged into a jet fluidized bed granulator, and granules were produced by using a liquid prepared by dissolving 40.0 g of hydroxypropylcellulose in 760 g of purified water and then were dried. Thus, an enteric granulated preparation having the composition given below was obtained.

| [Ingredients] | [Weight (mg) in 500 mg granulated preparation] |
|---|---|
| Sodium rabeprazole | 10.0 |
| Calcium silicate | 5.0 |
| Sodium hydroxide | 1.0 |
| Polyvinyl alcohol copolymer | 12.0 |
| Talc | 36.0 |
| Methacrylic acid copolymer LD | 46.5 |
| Triethyl citrate | 4.5 |
| D-mannitol | 365.0 |
| Hydroxypropylcellulose | 20.0 |

### Example 8

Into the same jet fluidized bed granulator as that used in Example 1 was charged 100.0 g of light anhydrous silicic acid. Subsequently, granules were produced by using a liquid prepared by dissolving 200.0 g of sodium rabeprazole in a liquid prepared by dissolving 20.0 g of sodium hydroxide in 480.0 g of purified water and then were dried. The granules were made uniform in size by being passed through a sieve of JIS 24 mesh (stabilized uniformized granules). As a result of the following operations performed in the same manner as in Example 7, an enteric granulated preparation having the composition given below was obtained.

| [Ingredients] | [Weight (mg) in 500 mg granulated preparation] |
|---|---|
| Sodium rabeprazole | 10.0 |
| Light silicic anhydride | 5.0 |
| Sodium hydroxide | 1.0 |
| Polyvinyl alcohol copolymer | 12.0 |
| Talc | 36.0 |
| Methacrylic acid copolymer LD | 46.5 |
| Triethyl citrate | 4.5 |
| D-mannitol | 365.0 |
| Hydroxypropylcellulose | 20.0 |

### Example 9

Into the same jet fluidized bed granulator as that used in Example 1 was charged 100.0 g of sodium carboxymethyl starch. Subsequently, granules were produced by using a liquid prepared by dissolving 200.0 g of sodium rabeprazole in a liquid prepared by dissolving 20.0 g of sodium hydroxide in 480.0 g of purified water and then were dried. The granules were made uniform in size by being passed through a sieve of JIS 24 mesh (stabilized uniformized granules). As a result of the following operations performed in the same manner as in Example 7, an enteric granulated preparation having the composition given below was obtained.

| [Ingredients] | [Weight (mg) in 500 mg granulated preparation] |
|---|---|
| Sodium rabeprazole | 10.0 |
| Sodium carboxymethylstarch | 5.0 |
| Sodium hydroxide | 1.0 |
| Polyvinyl alcohol copolymer | 12.0 |
| Talc | 36.0 |
| Methacrylic acid copolymer LD | 46.5 |
| Triethyl citrate | 4.5 |
| D-mannitol | 365.0 |
| Hydroxypropylcellulose | 20.0 |

Test Example 1 (Measurement of the content of a granulated preparation and a tablet in an accelerated test)
Samples obtained from the granulated preparations obtained in Examples 1 to 3 by applying aluminum heat-seal wrapping and further aluminum bag packaging (with a desiccating agent), samples obtained from the tablets of Examples 4 to 6 by applying PTP packaging and further aluminum bag packaging (with a desiccating agent), and a commercially available tablet preparation containing 10 mg of sodium rabeprazole were stored at a temperature of 40°C and a relative humidity of 75%. At one month and at three months from the start of the storage, the sodium rabeprazole contents in the respective granulated preparations and the respective tablets were measured by high performance liquid chromatography and the results given in Table 1 were obtained.

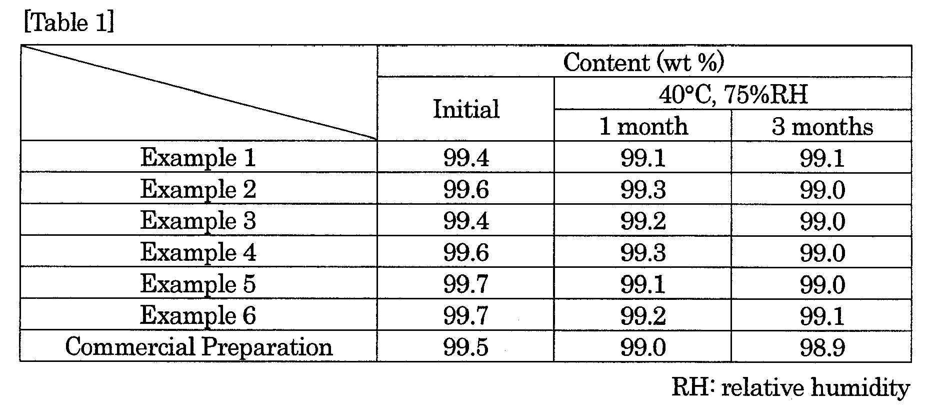

The results given in Table 1 showed that like the commercially available preparation, the preparations of Examples 1 to 6 according to the present invention were able to hold sodium rabeprazole stably.

Test Example 2 (Measurement of the content of a granulated preparation and a tablet in a severe test)
For the preparations (tablets and granulated preparations) of Examples 5 to 9 and a commercially available tablet preparation containing 10 mg of sodium rabeprazole, samples stored at a temperature of 40°C and a relative humidity of 75% in a laboratory dish-opened state and samples stored at a temperature of 60°C and a relative humidity of 75% in a glass bottle-sealed state were measured for their sodium rabeprazole contents in the respective tablets by high performance liquid chromatography one week after the start of the storage, and the results given in Table 2 were obtained.

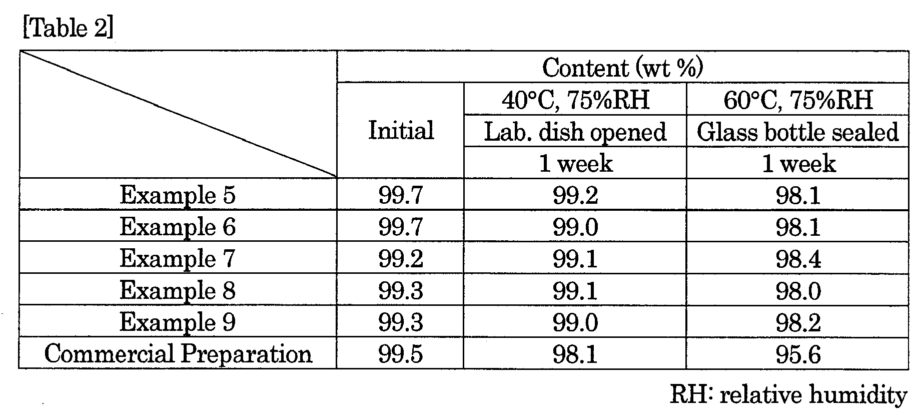

The results given in Table 2 showed that the preparations of Examples 5 to 9 according to the present invention were able to hold sodium rabeprazole more stably under severe conditions than the commercially available preparation.

### INDUSTRIAL APPLICABILITY

According to the present invention, the stability in quality can be secured by performing aqueous stabilizing treatment for a product containing an unstable substance. Moreover, because of no use of organic solvents, such as ethanol, there are no problems, such as explosion and air pollution, and on-the-job injuries of workers can be prevented, so that the production efficiency can be improved. Therefore, the present invention is high in industrial utility value.

## Claims

1. A method for producing a granulated preparation, which comprises supplying particles of a stabilizing agent or an excipient to a fluidized bed granulator, and spraying a liquid in which an unstable substance has been dissolved or suspended in an aqueous solution or aqueous suspension of the stabilizing agent while keeping a fluidized state.

2. The method for producing a granulated preparation according to claim 1, wherein the stabilizing agent is an alkaline substance and the unstable substance is a substance unstable under a neutral or acidic condition.

3. The method for producing a granulated preparation according to claim 1 or 2, wherein the stabilizing agent is a hydroxide, oxide or carbonate of an alkali metal or alkaline earth metal, and the unstable substance is a proton pump inhibitor.

4. The method for producing a granulated preparation according to any one of claims 1 to 3, wherein the stabilizing agent is sodium hydroxide, magnesium oxide, potassium carbonate, sodium carbonate, or potassium hydroxide and the unstable substance is sodium rabeprazole, omeprazole, or lansoprazole.

5. The method for producing a granulated preparation according to any one of claims 1 to 4, wherein the amount of the stabilizing agent in the aqueous solution or the aqueous suspension of the stabilizing agent is 0.01 to 10% by weight relative to the granulated preparation.

6. The method for producing a granulated preparation according to any one of claims 1 to 5, wherein the concentration of the stabilizing agent in the aqueous solution or the aqueous suspension of the stabilizing agent is 0.1 to 33% by weight.

7. The method for producing a granulated preparation according to any one of claims 1 to 6, wherein a surface of the excipient has been subjected to stabilizing treatment with a stabilizing agent.

8. The method for producing a granulated preparation according to any one of claims 1 to 7, wherein the excipient is lactose, crystalline cellulose, corn starch, potato starch, partially pregelatinized starch, D-mannitol, white soft sugar, sucrose, glucose, light silicic anhydride, calcium silicate, or sodium carboxymethylstarch.

9. A method for producing a granulated preparation, which comprises forming a monolayer or multilayer film by using an aqueous macromolecular film-forming agent liquid in which a stabilizing agent or another additive has been dissolved or suspended, on the surfaces of granules prepared by the granulation method according to any one of claims 1 to 8, and subsequently coating the granules with film-forming agent for elution control.

10. The method for producing a granulated preparation according to claim 9, wherein the macromolecular compound of the macromolecular film forming agent is at least one member selected from the group consisting of hydroxypropylcellulose, hydroxypropylmethylcellulose, polyvinyl pyrrolidone, polyvinyl alcohol, polyvinyl alcohol copolymer, aminoalkyl methacrylate copolymer (E, RS), methacrylic acid copolymer (L, LD, S), methylcellulose, hydroxypropyl, methylcellulose phthalate, hydroxypropylmethylcellulose acetate succinate, and ethylcellulose compounds.

11. A method for producing a tablet, which comprises mixing granules prepared by the granulation method according to claim 9 or 10 with another additive, and compressing them to make tablets.

12. A method for producing a tablet, which comprises mixing granules prepared by the granulation method according to any one of claims 1 to 8 with another additive, compressing them, then forming a monolayer or multilayer film by using an aqueous macromolecular film-forming agent liquid in which a stabilizing agent or another additive has been dissolved or suspended, and subsequently coating a tablet with a film-forming agent for elution control.
